# FASCICULE DE BREVET EUROPEEN

(11) **EP 0 988 092 B1**
(45) Date de publication et mention de la délivrance du brevet: **11.06.2008**
(21) Numéro de dépôt: 98930828.3
(22) Date de dépôt: 11.06.1998
(51) Int. Cl.: A61N 7/02, A61B 17/22

(54) **APPLICATEUR INTRATISSULAIRE ULTRASONORE POUR L'HYPERTHERMIE**
INTERSTITIELLER ULTRASCHALL-THERMOTHERAPIE-APPLIKATOR
ULTRASOUND INTRATISSULAR APPLICATOR FOR THERMOTHERAPY

(30) Priorité: 11.06.1997 FR 9707529
(43) Date de publication de la demande: 29.03.2000
(73) Titulaire: INSTITUT NATIONAL DE LA SANTE ET DE LA RECHERCHE MEDICALE (INSERM), 75654 Paris Cédex 13 (FR)
(72) Inventeur: LAFON, Cyril, F-19130 Objat (FR); CHAPELON, Jean-Yves, F-69100 Villeurbanne (FR); CATHIGNOL, Dominique, F-69740 Genas (FR); PRAT, Frédéric, F-91140 Villebon sur Yvette (FR)
(74) Mandataire: Blanchard, Eugène Gilles
(86) Numéro de dépôt international: PCT/FR1998/001212
(87) Numéro de publication internationale: WO 1998/056462

(56) Documents cités:
- EP-A- 0 643 982
- WO-A-95/02994
- US-A- 4 938 216
- US-A- 5 402 792
- US-A- 5 620 479
- HAND ET AL.: "AN ULTRASOUND LINEAR ARRAY FOR USE IN INTRACAVITARY APPLICATORS FOR THERMOTHERAPY OF PROSTATIC DISEASES" IEEE 1993 ULTRASONICS SYMPOSIUM, 31 octobre 1993, pages 1225-1228, XP000475278 BALTIMORE, US

## Description

### DOMAINE TECHNIQUE :

La présente invention est relative aux dispositifs mis en oeuvre pour le traitement par hyperthermie localisée de tumeurs plus généralement malignes.

Dans le domaine technique ci-dessus, il est connu de traiter les tumeurs par application d'une élévation localisée de température. De nombreuses publications évoquent la méthode consistant à élever la température de la zone concernée aux alentours de 45 ° C afin de rendre les tissus sensibles aux méthodes plus traditionnelles de traitement, comme la radiothérapie et la chimiothérapie.

Depuis quelque temps, il a été proposé de traiter les tumeurs en chauffant les tissus à des températures plus importantes, voisines de 80 °C, dans le but de réaliser une nécrose de coagulation. Le but visé par ce développement n'est donc plus la préparation des tissus à une sensibilité accrue aux méthodes de traitement traditionnel mais bien une tentative de destruction des tissus par nécrose localisée et contrôlée.

Pour mener à bien une telle méthode nouvelle de traitement, il a été proposé des applicateurs comportant une tête chargée d'apporter de la chaleur dans les tissus concernés en mettant en oeuvre des moyens de production qui ont fait appel à plusieurs principes physiques. Il peut être cité à cet égard les micro-ondes, les ultrasons, les résistances chauffantes, les lasers, etc...

### TECHNIQUE ANTERIEURE :

Pour entreprendre un tel traitement, la technique antérieure a connu des propositions de deux ordres.

Il a, en effet, été proposé des applicateurs hyperthermiques externes placés en surface pour un traitement des tumeurs sous-cutanées ou à faible profondeur dermique ou encore directement accessibles à partir de la peau.

Il a aussi été proposé, pour le traitement des tumeurs inaccessibles par voie externe, des applicateurs dits intratissulaires qui sont conçus pour être amenés au sein de la zone à traiter par voie endoscopique ou endocanalaire.

La présente invention vise spécifiquement les dispositifs de second ordre et concerne donc les applicateurs intratissulaires et, plus particulièrement, des applicateurs mettant en oeuvre des ultrasons pour chauffer la ou les zones à traiter par voie interne.

Les publications qui se référent à de tels matériels font état d'applicateurs produisant une enveloppe cylindrique ou sphérique de chaleur en raison de la forme de la partie active de l'applicateur.

Ainsi, le brevet US 5 620 479 décrit un applicateur intratissulaire comprenant une tête d'application de forme sensiblement cylindrique. La tête d'application comporte plusieurs transducteurs ultrasonores cylindriques reliés à un générateur électrique par des lignes conductrices. La face externe ou émettrice des transducteurs est en relation avec la paroi de la tête d'application qui est transparente aux ultrasons, tandis que la face interne opposée à la face émettrice est en relation avec un volume d'air.

De telles têtes d'application se caractérisent par le fait que les transducteurs cylindriques produisent des ondes ultrasonores divergentes, de sorte que la quantité de chaleur produite et donc l'élévation de température, décroît très vite au fur et à mesure de l'éloignement de la source productrice. L'efficacité de traitement en profondeur dans une direction donnée est donc relativement limitée, à moins de faire intervenir des puissances plus importantes qui ont pour inconvénient de faire naître, par l'augmentation de l'énergie fournie par l'applicateur, une vaporisation des tissus au voisinage et/ou au contact de la tête.

Il en résulte une difficulté de contrôle en profondeur qui se trouve, par ailleurs, rendue encore plus incertaine en raison de la vascularisation plus ou moins prononcée, variable et aléatoire, des tissus concernés.

Un autre inconvénient tenant aux applicateurs jusqu'à présent connus, réside dans le fait que les têtes cylindriques ou sphériques ne permettent pas d'élire une direction privilégiée de traitement et, en conséquence, de provoquer des nécroses de coagulation très précisément délimitées dans un champ connu impliquant le respect de l'intégrité des tissus environnants qui ne doivent pas être nécrosés.

Un autre inconvénient des applicateurs connus, réside dans le fait que la dispersion thermique que les têtes d'application produisent par leur diffusion omnidirectionnelle impose, pour la compenser, de procéder à des applications de longue durée, donnant lieu à traitements relativement lourds, coûteux à conduire et dépendant considérablement de la perfusion des tissus environnants.

Pour apporter une solution au problème de la dispersion de l'énergie acoustique émise, liée à la divergence des ondes ultrasonores engendrées par un transducteur cylindrique, l'art antérieur a proposé de mettre en oeuvre des applicateurs intratissulaires comportant un ou plusieurs transducteurs engendrant des ondes ultrasonores focalisées vers une ou plusieurs cibles.

Un brevet US 5 402 792 présente un applicateur intratissulaire comportant une tête de forme sensiblement cylindrique délimitant une cavité dans laquelle est disposé un transducteur dont la face émettrice est constituée par une lentille acoustique concave présentant deux foyers acoustiques. Par ailleurs, la face émettrice du transducteur est recouverte à distance d'une membrane étanche perméable aux ultrasons, qui délimite une cavité remplie d'eau dégazée pour assurer un couplage acoustique entre la face émettrice et la membrane.

Un autre brevet US 4,938,216 décrit un applicateur ultrasonore comprenant, dans une tête d'application, un transducteur ultrasonore plan, associé à une lentille qui concentre les ondes ultrasonores en un foyer linéaire.

Une demande WO 95/02944 décrit, quant à elle, un dispositif dont le transducteur ultrasonore est associé à une lentille convergente à foyer ponctuel dont la distance focale est réglable.

La mise en oeuvre de telles ondes ultrasonores convergentes permet effectivement la concentration de l'énergie acoustique émise dans des régions très localisées et d'extension réduite. Toutefois, compte tenu de cette faible extension, il est impératif d'assurer un positionnement très précis de l'applicateur par rapport à la zone à traiter. Or, une telle précision n'est pas toujours possible selon la région du corps du patient à traiter.

De plus, les ondes ultrasonores émises par un transducteur focalisé sont, tout d'abord, convergentes jusqu'au foyer puis, ensuite, divergentes à partir du foyer, de sorte que le rayonnement acoustique n'est pas homogène et que la quantité de chaleur produite décroît très vite au fur et à mesure de l'éloignement par rapport au foyer et dans une direction opposée au transducteur, comme cela est le cas pour un transducteur cylindrique.

La demande WO 95/02944 décrit aussi un applicateur selon le préambule de la revendication 1.

### EXPOSE DE L'INVENTION :

La présente invention vise à remédier à l'ensemble des inconvénients ci-dessus en proposant un nouvel applicateur ultrasonore, de préférence, intratissulaire d'application hyperthermique localisée dont la conception est choisie pour rendre possible un apport de chaleur dans une direction privilégiée tout en assurant une bonne pénétration de l'énergie acoustique combinée à une distribution homogène de celle-ci et en permettant un traitement omnidirectionnel dans le cas nécessaire.

Un autre objet de l'invention est de produire un applicateur ultrasonore qui puisse être utilisé facilement par voie endoscopique ou endocanalaire, en étant introduit directement dans une voie naturelle ou par l'intermédiaire du canal opérateur d'un endoscope.

Un applicateur ultrasonore pour atteindre les objectifs ci-dessus est défini dans la revendication 1.

Diverses autres caractéristiques ressortent de la description faite ci-dessous en référence aux dessins annexés qui montrent, à titre d'exemples non limitatifs, des formes de réalisation de l'objet de l'invention.

### BREVE DESCRIPTION DES DESSINS :

La **fig. 1** est une coupe-élévation de l'applicateur conforme à l'invention.
Les **fig. 2** et **3** sont des coupes transversales prises selon les lignes II-II et III-III de la **fig. 1**.
La **fig. 4** est une vue de dessus, partie en coupe, d'un autre exemple de réalisation de l'applicateur.
Les **fig. 5** et **6** sont des coupes transversales prises selon les lignes V-V et VI-VI de la **fig. 4****.**
La **fig. 7** est une coupe prise selon la ligne brisée VII-VII de la **fig. 5****.**

### MEILLEURE MANIERE DE REALISER L'INVENTION :

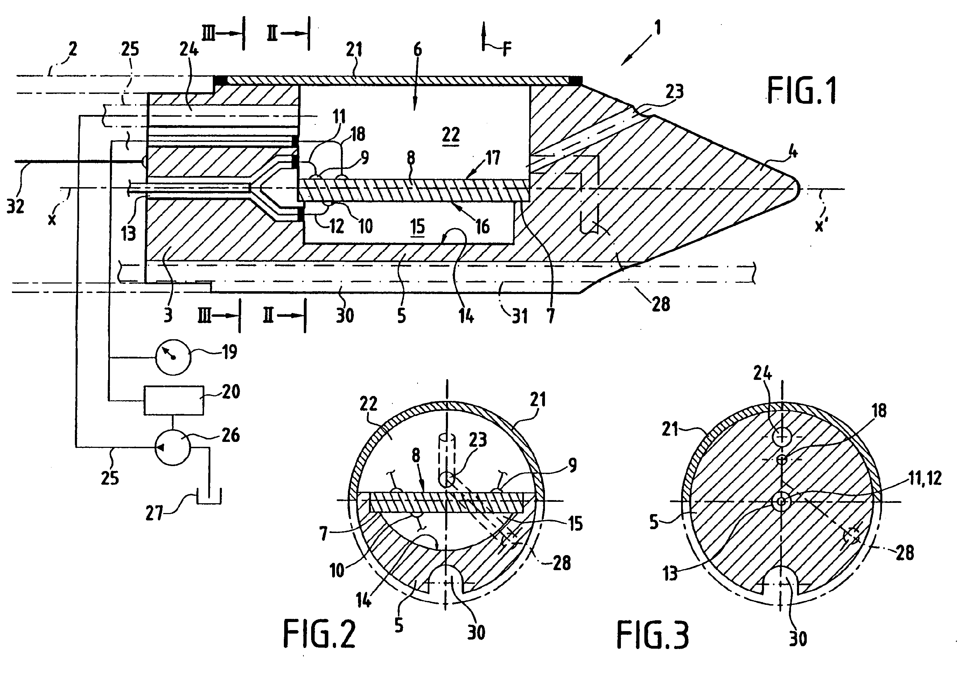

Selon les **fig. 1** à **3****,** l'applicateur ultrasonore intratissulaire comprend une tête d'application **1** qui est, de préférence mais non exclusivement, réalisée en une matière non ferromagnétique, de manière à pouvoir être mise en oeuvre en complémentarité d'installations de traitement ou de contrôle parallèles telles que celle faisant intervenir le principe de la résonnance magnétique nucléaire.

La tête **1** est portée par un élément tubulaire **2** qui est ici constitué par un élément rigide adapté de toute façon appropriée étanche sur la partie **3** de la tête **1,** considérée comme arrière par rapport à un embout avant **4,** dit de pénétration. Il doit être considéré que l'élément tubulaire **2** pourrait être entièrement constitué, ou pour partie complété, par une gaine à caractère souple, pour faciliter un cheminement contrôlé, endoscopique ou endocanalaire. A titre d'exemple, une telle gaine pourrait être constituée avantageusement par une succession d'enroulements co-axiaux filaires en spires contiguës à partir d'un fil de section circulaire ou polygonale.

La tête **1** comprend un corps **5** formant les parties arrière **3** et avant **4** entre lesquelles il présente un chambrage **6** qui est au moins ouvert sur une partie de la périphérie du corps **5.** Dans l'exemple d'application illustré, le corps **5** est de forme générale préférentiellement cylindrique et la partie **4** présente alors avantageusement une forme ogivale, tronc-pyramidale ou conique, bien qu'une forme sphérique ou semi-sphérique puisse aussi être retenue.

Le chambrage **6** est réalisé pour délimiter, sensiblement au milieu de sa profondeur dans l'exemple représenté, un épaulement ou siège **7** sur lequel peut être monté, adapté, immobilisé avec étanchéité, un transducteur ultrasonore plan **8,** de forme générale rectangulaire dont la longueur est orientée dans le sens de l'axe longitudinal x-x', du corps **5** et, plus particulièrement, dans l'exemple illustré, parallèlement à cet axe.

Le transducteur ultrasonore **8** est relié par des connexions de surface **9** et **10** à deux fils d'alimentation **11** et **12,** qui sont engagés à travers la partie arrière **3** par l'intermédiaire d'un perçage **13** traversant s'ouvrant dans le chambrage **6.** Les fils d'alimentation **11** et **12** sont raccordés à une source électrique non représentée, capable d'appliquer au transducteur des fréquences de l'ordre de 5 à 10 MHz. Le transducteur **8** peut alors être alimenté par un signal électrique monofréquence ou multifréquences selon la nature de l'émission ultrasonore souhaitée.

Le montage étanche du transducteur **8** sur l'épaulement **7** et la position de ce dernier par rapport au fond **14** du chambrage **6** sont définis de manière qu'après adaptation du transducteur, celui-ci délimite avec le fond **14** une chambre **15** qui est remplie d'air de manière à constituer un moyen de non-propagation ultrasonore à partir de la grande face **16** arrière du transducteur **8.**

Il en résulte l'existence d'une face d'émission privilégiée plane constituée par la grande face avant **17** qui est celle orientée vers l'ouverture du chambrage 6 par rapport à la périphérie du corps **5.** De manière préférée, la face d'émission plane présente des dimensions suffisantes pour émettre, lors du fonctionnement du transducteur, des ondes ultrasonores sensiblement planes qui ne divergent pas au voisinage du transducteur et de l'applicateur.

Le transducteur ultrasonore **8** est associé à des moyens d'appréciation de sa montée en température. De tels moyens sont, dans l'exemple illustré, constitués par un thermocouple **18** qui traverse de façon étanche la partie arrière **3** de la tête **1** pour être raccordé à un témoin visuel **19,** tel qu'un indicateur de température. Le thermocouple **18** est de préférence aussi, couplé à un dispositif régulateur **20** dont la fonction apparaît dans ce qui suit. Il pourrait être retenu de remplacer le thermocouple **18** par un moyen équivalent par exemple, un moyen de mesure de la variation de la capacité électrique du transducteur ultrasonore en fonction de sa température.

Le chambrage **6** est fermé dans sa partie s'ouvrant à la périphérie du corps **5** par l'intermédiaire d'une membrane **21** constituée par une feuille en une matière ne faisant intervenir qu'un faible coefficient d'atténuation ultrasonique. Avantageusement, cette membrane **21** est constituée par une feuille en polyéthylène basse pression d'une épaisseur de 12 µm rapportée sur le corps **5** par l'intermédiaire d'une colle appropriée telle qu'une colle du type cyanocrylate.

La membrane **21** peut être constituée par un segment tubulaire entourant complètement le corps **5** ou par un segment sectoriel couvrant l'ouverture du chambrage **6.**

La présence de la membrane **21** permet de délimiter dans le chambrage **6,** avec la face **17** du transducteur **8,** une cavité **22** fermée de façon étanche et qui est en communication, d'une part, avec un trou **23** pratiqué dans la partie **4** au niveau de la surface extérieure de laquelle il s'ouvre et, d'autre part, avec un conduit **24** dit d'amenée, traversant la partie arrière **3.** Le conduit **24** est raccordé à un élément tubulaire **25** faisant partie d'un circuit de circulation d'un fluide de refroidissement fourni par une pompe **26** à partir d'une réserve **27.** Le fluide de refroidissement peut être de différente nature, tout en étant choisi de manière à remplir une fonction de couplage ultrasonore entre le transducteur **8** et la membrane **21** en occupant toujours complètement la cavité **22.** A titre d'exemple préférentiel, un tel fluide est constitué par de l'eau dégazée.

Le fonctionnement de la pompe **26,** au moins pour ce qui concerne le débit fourni, est placé sous la dépendance du régulateur **20** appréciant la montée en température du transducteur ultrasonore **8.**

Le circuit de circulation de fluide pourrait faire intervenir, outre le circuit aller **25,** un circuit de retour **28** dit aussi "de reprise", ménagé dans la partie avant **4,** à la place du trou d'évacuation **23.** Un tel circuit de reprise pourrait alors être raccordé directement à la source **27.**

En règle générale, la partie de l'élément tubulaire **2** faisant suite à la partie arrière **3** du corps de tête **1** est remplie d'une matière d'occupation, d'étanchéification et de comblement visant à isoler, contenir, maintenir, confiner et protéger les lignes d'asservissement qui sont constituées par les fils d'alimentation **11** et **12,** par le thermocouple **18** et par le ou les circuits **25.**

L'applicateur décrit ci-dessus peut être introduit en raison de sa forme même à proximité, voire dans une tumeur à traiter, par voie interne, i.e. par voie endoscopique ou intracanalaire, de façon à appliquer, au sein de cette zone, une montée en température qui est créée par l'absorption des ultrasons dans le milieu absorbant ou les tissus, situés en regard de la face d'émission, après le raccordement du transducteur **8** à la source électrique.

En raison de la présence du coussin d'air occupant la chambre **15,** le fonctionnement du transducteur **8** se traduit par une émission ultrasonore uniquement à partir de la face d'émission **17** qui produit un champ proche, non divergent, traversant la cavité **22** et la membrane **21** selon une direction de propagation normale au plan de la face d'émission **17.** La montée en température du transducteur ultrasonore **8,** due au fait que son rendement électro-acoustique est inférieur à 1, est appréciée par l'intermédiaire du moyen **18** qui fournit une information au régulateur **20** permettant de contrôler les conditions de fonctionnement de la pompe **26** pour fournir, à l'intérieur de la cavité **22** toujours pleine, un débit de fluide de refroidissement convenable, évacué par le trou **23** par perfusion naturelle dans les tissus ou par reprise par le conduit **28.**

De cette manière, il est possible d'émettre un champ puissant sans provoquer une montée en température locale élevée qui pourrait autrement être de nature à produire ou provoquer une rupture de la membrane par échauffement et/ou la génération de microbulles d'ébullition entre la membrane **21** et le tissu et qui auraient pour effet d'interrompre la propagation des ultrasons avec pour conséquence la destruction du transducteur ultrasonore.

L'émission d'un champ proche de direction privilégiée sensiblement perpendiculaire à la face d'émission, comme l'indique la flèche **F,** permet d'engager un traitement sélectif, précis et localisé d'une tumeur par approche centralisée ou à distance, laquelle tumeur peut toutefois être aussi traitée de façon plus complète par application volumique de chaleur en soumettant le transducteur **8** à une rotation sur une plage angulaire sectorielle ou totale. A cette fin, l'applicateur selon l'invention comporte des moyens pour déplacer en rotation le transducteur. Selon l'exemple illustré, ces moyens de rotation sont constitués par l'élément tubulaire **2** qui permet l'entraînement en rotation de la tête d'application **1** sur son axe **x-x'** et, par suite, la rotation du transducteur **8.** De manière préférée, l'élément tubulaire **2** présente un coefficient de résistance à la torsion élevé pour permettre un entraînement en rotation convenable de la tête d'application.

Les moyens pour déplacer en rotation le transducteur pourraient également être constitués par des moyens moteurs ou de transmission permettant d'assurer une rotation du transducteur seul, sans déplacement conjoint de la tête d'application. De manière préférée mais non exclusive, la rotation du transducteur est effectuée autour d'un axe parallèle à un grand axe de la face d'émission. Par ailleurs, il doit être noté que la rotation du transducteur peut être continue pendant la durée d'émission en étant alternée ou non, mais également séquentielle en faisant intervenir une rotation pas à pas du transducteur, de manière à chauffer successivement des zones contiguës.

Il peut aussi être procédé à un déplacement alternatif ou non, rectiligne et d'amplitude variable sur l'axe x-x' de la tête **1** pour produire une nécrose d'un volume plus important de tissu. Un tel déplacement en translation de la tête peut également être combiné à un mouvement de rotation du transducteur.

Ainsi, par les moyens selon l'invention de contrôle et de régulation de température à partir de la face d'émission **17** du transducteur **8** et en raison de l'émission d'un champ proche directionnel selon la flèche F, il devient possible de procéder au traitement par hyperthermie localisée de tumeurs de faible volume ou non, à distance plus ou moins importante de la tête **1** et de conduire, par ce moyen, des traitements de tumeurs qui seraient ordinairement considérées, par leur localisation spécifique, inaccessibles à toute possibilité d'application hyperthermique localisée.

En adoptant un transducteur de 10 mm sur 3 mm et d'épaisseur λ/2, λ étant la longueur d'onde, réalisé en PZT 462 et susceptible de fonctionner sous des fréquences de 3 à 20 MHz, il est possible de réaliser un applicateur d'un diamètre extérieur voisin de 3,6 mm en faisant intervenir des circuits **25** de diamètre égal à 0,8 mm susceptibles d'entretenir une circulation de fluide de refroidissement dans la cavité **22** à raison de 7 ml/min.

De tels moyens sont favorables à une application de température élevée pendant des durées courtes qui sont néanmoins suffisantes pour atteindre une nécrose de coagulation.

En effet, la mise en oeuvre d'un transducteur présentant une face d'émission plane permet la production d'ondes ultrasonores sensiblement planes qui assurent une diffusion homogène et profonde de l'énergie acoustique dans le milieu absorbant.

De plus, compte tenu de la nature plane des ondes émises par un transducteur plan, ce dernier offre un rendement puissance acoustique émise par rapport à la puissance fournie au transducteur supérieure à celui offert par un transducteur cylindrique selon l'art antérieur.

Ainsi, dans le cadre d'un essai *in vivo* sur un foie de porc, un applicateur conforme à l'invention, mettant en oeuvre un transducteur plan de dimension 10 mm x 3 mm alimenté par un signal électrique d'une fréquence de 10 MHz, de manière à rayonner au niveau de sa surface d'émission, une puissance de 14 Watts/cm², permet d'obtenir après une durée d'émission de 20 secondes, une lésion en regard de la face émettrice présentant une profondeur de 10 à 12 mm.

En revanche, dans le cadre d'un essai *in vivo* identique, un applicateur présentant des dimensions externes identiques à celles du précédent mais mettant en oeuvre un transducteur cylindrique selon l'art antérieur de diamètre externe 3 mm et de longueur 10 mm, alimenté par un signal électrique d'une fréquence de 10 MHz, de manière à rayonner au niveau de sa surface d'émission une puissance de 14 Watts/cm², permet d'obtenir, après une durée d'émission de 20 secondes, une lésion, en regard de la face d'émission cylindrique du transducteur, présentant une profondeur de 2 à 3 mm seulement.

Il apparaît donc que toute chose étant égale par ailleurs, la mise en oeuvre d'un transducteur plan selon l'invention permet avantageusement d'augmenter la profondeur de lésion d'un facteur 3,33 à 6 par rapport à un transducteur cylindrique.

De même, en utilisant un applicateur selon l'invention, tel que celui décrit ci-dessus mis en oeuvre dans les mêmes conditions d'alimentation électrique et de puissance, il est possible en déplaçant en rotation la tête d'application autour de son axe, d'engendrer, avec une durée d'émission de 7 à 8 mn, une lésion cylindrique présentant un diamètre extérieur de 10 à 12 mm.

Or, un applicateur selon l'art antérieur avec un transducteur cylindrique, tel que celui décrit ci-dessus, ne permet d'obtenir une lésion cylindrique présentant un diamètre extérieur de 10 à 12 mm qu'avec une durée d'émission de 18 à 20 mn.

Il apparaît clairement que la mise en oeuvre d'un transducteur plan selon l'invention permet avantageusement, toute chose étant égale par ailleurs, une réduction de la durée d'émission et donc de chauffage d'un facteur 1,5 à 2 par rapport à un transducteur cylindrique.

Les caractéristiques constructives et fonctionnelles ouvrent largement la possibilité d'utiliser l'applicateur par voie interne, soit par voie endoscopique, soit encore par voie intra-canalaire en constituant, pour partie au moins, l'élément 2 sous la forme d'une gaine souple, soit encore par application percutanée à partir d'un élément 2 tubulaire rigide.

Pour faciliter le cheminement intra-canalaire ou à l'intérieur d'un guide opérateur endoscopique, il est avantageusement prévu de ménager dans le corps **5** une rainure ou un passage **30** permettant l'engagement d'un fil guide **31** favorisant l'évolution intra-canalaire de la tête **1,** notamment lorsque le canal investi est de section relativement faible et connaît des variations d'orientation à faible rayon de courbure. Il est également avantageusement prévu de lier le corps **5** par sa partie arrière **3** à un élément filaire de traction **32** dont la fonction est de permettre, notamment lorsque la gaine **2** est constituée sous la forme d'un enroulement filaire hélicoïdal, d'exercer sur l'applicateur un effort de traction pour favoriser son extraction de l'intérieur du canal ou du guide opérateur endoscopique investi.

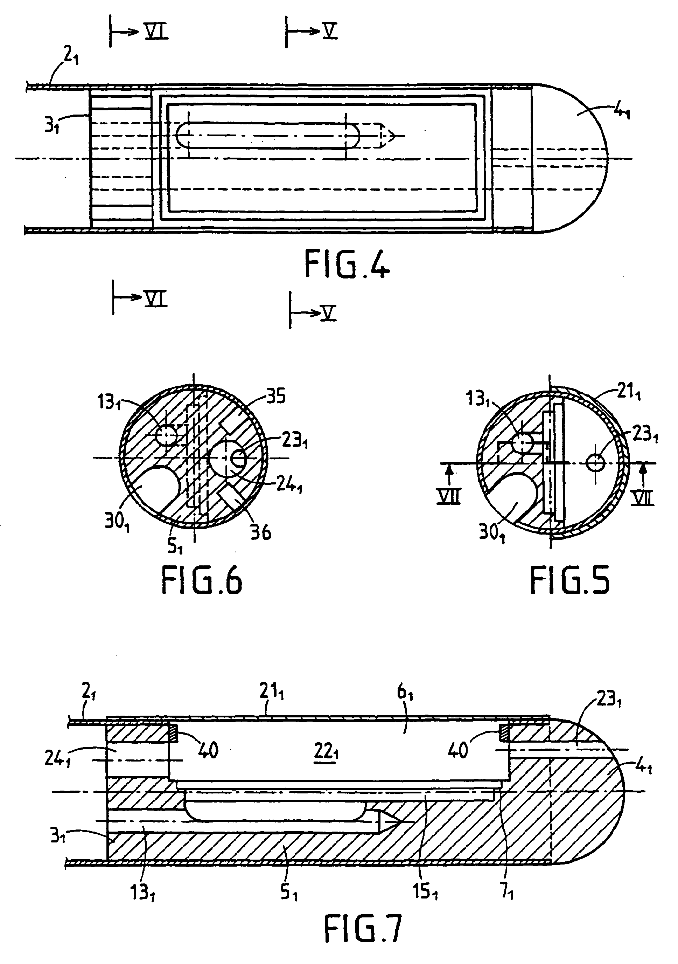

Les **fig. 4** à **7** montrent une forme préférée de réalisation industrielle de la tête **1** qui comporte un corps **5₁** inséré dans un élément tubulaire **2₁** dont la longueur au-delà de la partie arrière **3₁**, est appropriée pour une fonction typiquement endoscopique ou une fonction endocanalaire par association avec une gaine souple déformable abritant comme dans l'exemple précédent les lignes d'établissement fonctionnel à distance de la tête **1.**

Dans un tel cas, bien que cela ne soit pas représenté, l'élément tubulaire **2₁** comporte une fenêtre ménagée en correspondance du chambrage **6₁**, une telle fenêtre étant fermée comme dans l'exemple précédent par une membrane **21₁**.

Dans l'exemple de réalisation selon les **fig. 4** à **7** les mêmes éléments constitutifs que ceux de l'exemple précédent sont désignés par les mêmes références affectées de l'indice₁. Dans cette forme de réalisation, il est avantageux de ménager, à partir de la périphérie du corps **5** des encoches ou rainures **35** et **36** réservées au passage des fils **11** et **12** en remplacement du perçage **13.**

Dans les exemples de réalisation ci-dessus il est considéré que la tête **1** est équipée d'un transducteur **8** dont la position est de préférence parallèle par l'une de ses grandes faces à l'axe longitudinal du corps **5₁**.

Il doit être considéré que la tête peut être équipée de plusieurs transducteurs **8** qui répondent à une semblable caractéristique en étant montés de même manière pour occuper toute ou partie de la périphérie du corps **5** en étant situés pour chacun d'eux en retrait de cette périphérie afin de ménager pour la face d'émission **17** une cavité de couplage par l'intermédiaire du fluide de refroidissement. Dans un tel cas, il doit aussi être retenu que chaque transducteur est associé à des moyens de non ou de faible propagation ultrasonique placés en relation avec la face arrière **16** afin de favoriser la seule émission d'un champ proche utile à partir de la face **17.**

Il peut donc être envisagé de faire comporter à la tête **1,** par exemple deux ou trois transducteurs.

Selon l'invention, il est également possible d'envisager de disposer un ou plusieurs transducteurs **8** pour qu'ils s'étendent de façon non parallèle à l'axe x-x' en présentant une inclinaison relativement à cet axe pour que le champ proche émis adopte une direction non normale à cet axe.

Il doit aussi être retenu que la sonde peut être pourvue d'au moins un transducteur **8** qui serait disposé perpendiculairement à l'axe x-x', en étant situé au voisinage de la partie avant **4.** Dans un tel cas, la membrane **21** pourrait alors être réalisée sous la forme d'un ballon entourant à distance le transducteur pour délimiter un volume de protection et de confinement d'un liquide de refroidissement et de couplage ultrasonore.

La **fig. 7** met en évidence qu'il peut être avantageux de faire comporter au corps **5,** par exemple au niveau du chambrage **6,** des repères radio-opaques **40** permettant d'assumer une fonction de repérage fluoroscopique lors d'un cheminement endoscopique ou endocanalaire.

Dans les exemples ci-dessus, l'applicateur comprend, de manière préférée, des moyens pour refroidir le transducteur. Toutefois, l'applicateur pourrait également ne pas comporter de tels moyens de refroidissement.

### POSSIBILITE D'APPLICATION INDUSTRIELLE :

L'utilisation de l'applicateur a été décrite en relation avec un traitement médical. Toutefois, l'applicateur pourrait être utilisé dans toute application industrielle pour laquelle il est nécessaire de chauffer localement, un milieu absorbant les ondes ultrasonores, dans une région située en profondeur par rapport à la surface extérieure de l'objet à traiter.

Par exemple, pour assurer la destruction par la chaleur d'un foyer parasitaire au sein d'un volume de bois ou d'un arbre dont la destruction n'est pas souhaitable, un procédé de chauffage conforme à l'invention consiste à percer un canal de faible diamètre pour le passage de l'applicateur, jusqu'au foyer. Un tel canal constitue, bien entendu, une voie interne artificielle pour la mise en place de l'applicateur.

Une fois l'applicateur conforme à l'invention placé en position, le transducteur est alimenté en courant électrique, d'une fréquence donnée, pendant une durée prédéterminée en fonction de la nature du foyer parasitaire et de l'étendue de ce dernier. Bien entendu, le transducteur peut être alimenté par un courant multifréquences.

Selon le volume du foyer parasitaire, le transducteur peut être déplacé en rotation pour chauffer une région angulaire plus étendue. Cette rotation peut être continue, en étant alternée ou non, pendant toute la durée du traitement, ou encore séquentielle pas à pas, de manière à chauffer successivement des zones contigües de la région à traiter.

Le procédé de chauffage pourrait également être utilisé pour le chauffage de résine thermodurcissable injectée en profondeur d'une pièce de bois à considérer dans le cadre, par exemple, de sa restauration.

L'invention n'est pas limitée aux exemples décrits et représentés car diverses modifications peuvent y être apportées sans sortir de son cadre.

## Revendications

1. - Applicateur ultrasonore pour le chauffage, par voie interne, d'un milieu absorbant les ultrasons, comprenant:
- une tête d'application **(1)** comportant au moins un transducteur ultrasonore plan **(8)** dont une face **(17),** dite d'émission, est recouverte à distance d'une membrane **(21)** étanche et transparente aux ultrasons, et dont une autre face, opposée à la face d'émission, est associée à des moyens **(15)** de non ou de faible propagation ultrasonore, la face d'émission étant plane pour émettre des ondes ultrasonores sensiblement planes non divergentes traversant la membrane selon une direction de propagation sensiblement normale à la face d'émission,
- des moyens **(11, 12)** pour relier à distance le transducteur à un générateur électrique,
- et des moyens **(22, 23, 24)** pour réaliser un couplage ultrasonore avec la membrane,
**caractérisé en ce que** :
- le transducteur ultrasonore **(8)** est immobilisé avec étanchéité sur un épaulement ou siège **(7)** délimité dans un chambrage **(6)** formé dans la tête d'application et ouvert sur une partie au moins de sa périphérie de manière à délimiter avec le fond **(14)** du chambrage **(6)** une chambre **(15)** remplie d'air constituant le moyen de non ou de faible propagation ultrasonore à partir de la face **(16)** opposée à la face d'émission du transducteur **(8),**
et **en ce qu'**il comporte des moyens de contrôle (18) et de régulation **(20)** de la température de la face d'émission **(17)** coopérant avec un circuit de circulation **(23, 24, 25, 26, 27)** d'un fluide de refroidissement sur la surface d'émission **(17)** du transducteur.

2. - Applicateur selon la revendication 1, **caractérisé en ce qu'**il comporte des moyens pour déplacer en rotation le transducteur plan.

3. - Applicateur selon la revendication 1 ou 2, **caractérisé en ce qu'**il comprend des moyens pour refroidir le transducteur.

4. - Applicateur selon l'une des revendications 1 à 3, **caractérisé en ce qu'**il comprend un élément tubulaire **(2)** portant ladite tête à une extrémité et abritant les lignes **(25, 11, 12, 18)** d'établissement fonctionnel à distance de la tête.

5. - Applicateur selon l'une des revendications 1 à 4, **caractérisé en ce que** la tête comporte au moins un transducteur ultrasonore plan dont la plus grande dimension est orientée dans le sens de l'axe longitudinal **(x-x')** de la tête.

6. - Applicateur selon la revendication 5, **caractérisé en ce que** la tête comporte au moins un transducteur ultrasonore plan dont la face d'émission est parallèle à l'axe longitudinal de la tête.

7. - Applicateur selon la revendication 5, **caractérisé en ce que** la tête comporte au moins un transducteur ultrasonore plan dont la face d'émission fait un angle avec l'axe longitudinal de la tête.

8. - Applicateur selon l'une des revendications 1 à 4, **caractérisé en ce que** la tête comporte au moins un transducteur ultrasonore plan dont la face d'émission est sensiblement normale à l'axe de la tête.

9. - Applicateur selon l'une des revendications 1 à 6 **caractérisé en que** la tête est réalisée en un matériau non ferro-magnétique.

10. - Applicateur selon l'une des revendications 1 à 9, **caractérisé en que** la tête comporte une membrane protectrice **(21)** réalisée sous la forme d'une enveloppe au moins partielle fermant une cavité d'application **(22)** présentée par la tête et dans laquelle est disposée le transducteur plan **(8).**

11. - Applicateur selon l'une des revendications 1 à 9, **caractérisé en ce que** la tête comporte une membrane protectrice réalisée sous la forme d'un ballon fermant une cavité d'application présentée par la tête et dans laquelle est disposé le transducteur plan.

12. - Applicateur selon la revendication 1, **caractérisé en ce que** le circuit de circulation de fluide de refroidissement comporte un conduit **(24)** raccordé à une ligne (25) d'amenée d'un fluide de refroidissement circulant sur la face d'émission du transducteur et occupant tout le volume délimité par la cavité **(22).**

13. - Applicateur selon la revendication 12, **caractérisé en ce que** la tête comporte un trou **(23)** d'évacuation vers l'extérieur du fluide de refroidissement.

14. - Applicateur selon la revendication 12, **caractérisé en ce que** la tête comporte un second conduit **(28)** dit de reprise raccordé à une ligne de retour du fluide de refroidissement.

15. - Applicateur selon la revendication 12, **caractérisé en ce que** les moyens de contrôle et de régulation de la température de la surface d'émission **(17)** du transducteur comportent des moyens **(18)** de mesure de la température du transducteur ainsi qu'à des moyens **(20)** de régulation de cette température.

16. - Applicateur selon l'une quelconque des revendications 1 à 15, **caractérisé en ce que** la tête comporte un conduit ou passage traversant **(31),** sensiblement parallèle à l'axe longitudinal de la tête et réservé à l'engagement d'un guide filaire de cheminement endocanalaire.

17. - Applicateur selon l'une quelconque des revendications 1 à 16, **caractérisé en ce que** la tête est raccordée à un élément filaire de traction **(32).**

18. - Applicateur selon l'une quelconque des revendications 1 à 17, **caractérisée en ce que** la tête est portée en bout d'un élément tubulaire constitué par une gaine souple.

19. - Applicateur selon l'une des revendications 1 à 18, **caractérisé en ce que** la tête porte au moins un repère radio opaque **(40)** assumant une fonction de repérage fluoroscopique.

20. **-** Applicateur selon la revendication 2, **caractérisé en ce que** la tête d'application possède une forme sensiblement cylindrique et que les moyens pour déplacer en rotation le transducteur sont constitués sous la forme sensiblement cylindrique de la tête d'application.

## Claims

1. An ultrasonic applicator for heating via an internal route, a medium which absorbs ultrasound, comprising:
- an application head (1) including at least one planar ultrasonic transducer (8), a so-called emission face (17) of which is remotely covered with a sealed membrane (21) transparent to ultrasound, and another face of which, opposite to the emission face, is associated with means (15) for non-or weak-propagation of ultrasound, the emission face being planar so as to emit non-divergent substantially plane ultrasonic waves passing through the membrane along a direction of propagation substantially normal to the emission face,
- means (11, 12) for remotely connecting the transducer to an electric generator,
- and means (22, 23, 24) for achieving ultrasonic coupling with the membrane,
**characterized in that**:
- the ultrasonic transducer (8) is sealably immobilized on a shoulder or seat (7) delimited in a recess (6) formed in the application head and open over at least one portion of its periphery so as to delimit with the bottom (14) of the recess (6) a chamber (15) filled with air forming the means for non-or weak-propagation of ultrasound from the face (16) opposite to the emission face of the transducer (8),
and **in that** it includes means for monitoring (18) and controlling (20) the temperature of the emission face (17) cooperating with a circuit (23, 24, 25, 26, 27) for circulating a coolant fluid on the emission surface (17) of the transducer.

2. The applicator according to claim 1, **characterized in that** it includes means for rotationally displacing the planar transducer.

3. The applicator according to claim 1 or 2, **characterized in that** it comprises means for cooling the transducer.

4. The applicator according to any of claims 1 to 3, **characterized in that** it comprises a tubular element (2) bearing said head at one end and remotely shielding the functional establishment lines (25, 11, 12, 18) from the head.

5. The applicator according to any of claims 1 to 4, **characterized in that** the head includes at least one planar ultrasonic transducer, the largest dimension of which is oriented in the direction of the longitudinal axis (x-x') of the head.

6. The applicator according to claim 5, **characterized in that** the head includes at least one planar ultrasonic transducer, the emission face of which is parallel to the longitudinal axis of the head.

7. The applicator according to claim 5, **characterized in that** the head includes at least one planar ultrasonic transducer, the emission face of which forms an angle with the longitudinal axis of the head.

8. The applicator according to any of claims 1 to 4, **characterized in that** the head includes at least one planar ultrasonic transducer, the emission face of which is substantially normal to the axis of the head.

9. The applicator according to any of claims 1 to 6, **characterized in that** the head is made in a non-ferromagnetic material.

10. The applicator according to any of claims 1 to 9, **characterized in that** the head includes a protective membrane (21) made as an at least partial cover closing an application cavity (22) presented head-on and in which the planar transducer (8) is positioned.

11. The applicator according to any of claims 1 to 9, **characterized in that** the head includes a protective membrane made as a balloon closing an application cavity presented head-on and in which the planar transducer is positioned.

12. The applicator according to claim 1, **characterized in that** the circuit for circulating a coolant fluid includes a conduit (24) connected to a line (25) for feeding coolant fluid circulating on the emission face of the transducer and occupying all the volume delimited by the cavity (22).

13. The applicator according to claim 12, **characterized in that** the head includes a hole (23) for discharging the coolant fluid outwards.

14. The applicator according to claim 12, **characterized in that** the head includes a second conduit (28), a so-called recirculation conduit, connected to a return line of the coolant fluid.

15. The applicator according to claim 12, **characterized in that** the means for monitoring and controlling the temperature of the emission surface (17) of the transducer include means (18) for measuring the temperature of the transducer as well as means (20) for controlling this temperature.

16. The applicator according to any of claims 1 to 15, **characterized in that** the head includes a conduit or through-passage (31) substantially parallel to the longitudinal axis of the head and reserved for engagement of a guide wire for following an endocanal route.

17. The applicator according to any of claims 1 to 16, **characterized in that** the head is connected to a pull-wire element (32).

18. The applicator according to any of claims 1 to 17, **characterized in that** the head is borne at the end of a tubular element formed by a flexible sheath.

19. The applicator according to any of claims 1 to 18, **characterized in that** the head bears at least one radio-opaque mark (40) fulfilling a fluoroscopic localization function.

20. The applicator according to claim 2, **characterized in that** the application head has a substantially cylindrical shape and that the means for rotationally displacing the transducer are formed under the substantially cylindrical shape of the application head.

## Patentansprüche

1. Ultraschallapplikator für das Erhitzen eines den Ultraschall absorbierenden Mediums auf innerem Weg, umfassend:
- einen Applikationskopf (1) mit wenigstens einem ebenen Ultraschallwandler (8), von dem eine Fläche (17), die sogenannte Sendefläche, im Abstand von einer dichten und ultraschalldurchlässigen Membran (21) bedeckt ist, und von dem eine andere, der Sendefläche gegenüberliegende Fläche Mitteln (15) für die Nicht- oder geringe Ultraschallausbreitung zugeordnet ist, wobei die Sendefläche eben ist, um im wesentlichen ebene, nicht divergente Ultraschallwellen auszusenden, welche die Membran in einer zur Sendefläche im wesentlichen normalen Ausbreitungsrichtung durchqueren,
- Mittel (11, 12) für die Fernverbindung des Wandlers mit einem Stromerzeuger,
- und Mittel (22, 23, 24) zur Herstellung einer Ultraschallkopplung mit der Membran,
**dadurch gekennzeichnet, daß**:
- der Ultraschallwandler (8) an einer Schulter oder einem Sitz (7), die bzw. der in einer Aussparung (6) begrenzt ist, die in dem Applikationskopf ausgebildet und über wenigstens einen Teil seines Umfangs offen ist, dicht festgelegt ist, so daß er mit dem Boden (14) der Aussparung (6) eine luftgefüllte Kammer (15) begrenzt, die das Mittel für die Nicht- oder geringe Ultraschallausbreitung von der der Sendefläche gegenüberliegenden Fläche (16) des Wandlers (8) aus bildet,
- und daß er Mittel zum Kontrollieren (18) und Regulieren (20) der Temperatur der Sendefläche (17) umfaßt, die mit einem Kreis (23, 24, 25, 26, 27) zum Zirkulieren eines Kühlfluids über die Sendefläche (17) des Wandlers zusammenwirken.

2. Applikator nach Anspruch 1, **dadurch gekennzeichnet, daß** er Mittel umfaßt, um den ebenen Wandler drehend zu bewegen.

3. Applikator nach Anspruch 1 oder 2, **dadurch gekennzeichnet, daß** er Mittel zum Kühlen des Wandlers umfaßt.

4. Applikator nach einem der Ansprüche 1 bis 3, **dadurch gekennzeichnet, daß** er ein röhrenförmiges Element (2) umfaßt, das den Kopf an einem Ende trägt und in dem die Leitungen (25, 11, 12, 18) zur funktionellen Fernversorgung des Kopfes untergebracht sind.

5. Applikator nach einem der Ansprüche 1 bis 4, **dadurch gekennzeichnet, daß** der Kopf wenigstens einen ebenen Ultraschallwandler umfaßt, dessen größte Abmessung in Richtung der Längsachse (XX') des Kopfes ausgerichtet ist.

6. Applikator nach Anspruch 5, **dadurch gekennzeichnet, daß** der Kopf wenigstens einen ebenen Ultraschallwandler umfaßt, dessen Sendefläche parallel zur Längsachse des Kopfes verläuft.

7. Applikator nach Anspruch 5, **dadurch gekennzeichnet, daß** der Kopf wenigstens einen ebenen Ultraschallwandler umfaßt, dessen Sendefläche mit der Längsachse des Kopfes einen Winkel bildet.

8. Applikator nach einem der Ansprüche 1 bis 4, **dadurch gekennzeichnet, daß** der Kopf wenigstens einen ebenen Ultraschallwandler umfaßt, dessen Sendefläche zur Achse des Kopfes im wesentlichen normal verläuft.

9. Applikator nach einem der Ansprüche 1 bis 6, **dadurch gekennzeichnet, daß** der Kopf aus einem nicht ferromagnetischen Material gefertigt ist.

10. Applikator nach einem der Ansprüche 1 bis 9, **dadurch gekennzeichnet, daß** der Kopf eine Schutzmembran (21) aufweist, die in Form einer wenigstens teilweisen Hülle ausgebildet ist, welche einen Applikationshohlraum (22) verschließt, den der Kopf aufweist und in dem der ebene Wandler (8) angeordnet ist.

11. Applikator nach einem der Ansprüche 1 bis 9, **dadurch gekennzeichnet, daß** der Kopf eine Schutzmembran aufweist, die in Form eines Ballons ausgebildet ist, der einen Applikationshohlraum verschließt, den der Kopf aufweist und in dem der ebene Wandler angeordnet ist.

12. Applikator nach Anspruch 1, **dadurch gekennzeichnet, daß** der Kühlfluidzirkulationskreis eine Leitung (24) umfaßt, die mit einer Leitung (25) zum Zuführen eines Kühlfluids verbunden ist, das über die Sendefläche des Wandlers zirkuliert und das gesamte durch den Hohlraum (22) begrenzte Volumen einnimmt.

13. Applikator nach Anspruch 12, **dadurch gekennzeichnet, daß** der Kopf ein Loch (23) zum Abführen des Kühlfluids zur Außenseite aufweist.

14. Applikator nach Anspruch 12, **dadurch gekennzeichnet, daß** der Kopf eine zweite Leitung (28), sogenannten Rücknahmeleitung aufweist, die mit einer Leitung zum Rückführen des Kühlfluids verbunden ist.

15. Applikator nach Anspruch 12, **dadurch gekennzeichnet, daß** die Mittel zum Kontrollieren und Regulieren der Temperatur der Sendefläche (17) des Wandlers Mittel (18) zum Messen der Temperatur des Wandlers sowie Mittel (20) zum Regulieren dieser Temperatur umfassen.

16. Applikator nach irgendeinem der Ansprüche 1 bis 15, **dadurch gekennzeichnet, daß** der Kopf eine(n) durchgehende(n) Leitung oder Durchgang (30) aufweist, die bzw. der zur Längsachse des Kopfes im wesentlichen parallel verläuft und für das Einführen eines Drahtleiters zur endokanalären Führung vorgesehen ist.

17. Applikator nach irgendeinem der Ansprüche 1 bis 16, **dadurch gekennzeichnet, daß** der Kopf mit einem Zugdrahtelement (32) verbunden ist.

18. Applikator nach irgendeinem der Ansprüche 1 bis 17, **dadurch gekennzeichnet, daß** der Kopf am Ende von einem von einer flexiblen Hülle gebildeten röhrenförmigen Element getragen wird.

19. Applikator nach irgendeinem der Ansprüche 1 bis 18, **dadurch gekennzeichnet, daß** der Kopf wenigstens einen radioopaken Marker (40) trägt, der eine Funktion zur fluoroskopischen Ortung übernimmt.

20. Applikator nach Anspruch 2, **dadurch gekennzeichnet, daß** der Applikationskopf eine im Wesentlichen zylindrische Form aufweist und daß die Mittel zum Drehbewegen des Wandlers unter der im wesentlichen zylindrischen Form des Applikationskopfes gebildet sind.
